# FASCICULE DE BREVET EUROPEEN

(11) **EP 0 622 109 B1**
(45) Date de publication et mention de la délivrance du brevet: **30.07.1997**
(21) Numéro de dépôt: 94400847.3
(22) Date de dépôt: 19.04.1994
(51) Int. Cl.: B01D 61/00, A61K 7/09, C07C 319/28

(54) **Procédé de désodorisation d'une formulation contenant au moins un composé porteur d'un groupe thiol**
Verfahren zum Desodorieren einer Formulierung,die mindestens eine Verbindung mit einer Thiolgruppe enthält
Process for deodorizing a formulation comprising at least one compound bearing a thiol group

(30) Priorité: 30.04.1993 FR 9305154
(43) Date de publication de la demande: 02.11.1994
(73) Titulaire: L'OREAL, 75008 Paris (FR)
(72) Inventeur: Burgaud, Hervé, F-77230 Dammartin en Goele (FR)
(74) Mandataire: Peuscet, Jacques

(56) Documents cités:
- EP-A- 0 488 665
- FR-A- 2 148 022
- FR-A- 2 606 027
- FR-A- 2 679 448
- US-A- 5 184 630
- PATENT ABSTRACTS OF JAPAN vol. 6, no. 261 (C-141)21 Décembre 1982 & JP-A-57 154 116 (TERUO TANIMURA) 22 Septembre 1982
- PATENT ABSTRACTS OF JAPAN vol. 8, no. 114 (C-225)26 Mai 1984 & JP-A-59 027 866 (RAION KK) 14 Février 1984
- DATABASE WPI Section Ch, Week 8836, Derwent Publications Ltd., London, GB; Class A08, AN 88-253215 & JP-A-63 183 064 (NAGATA) 28 Juillet 1988
- WPI/DERWENT, AN-88-068356 & JP63023708
- WPI/DERWENT, AN-88-356136 & JP63264139

## Description

La présente invention concerne un procédé de désodorisation d'une formulation contenant au moins un composé porteur d'un groupe fonctionnel thiol (-SH) et la formulation désodorisée ainsi obtenue.

Les composés organiques porteurs de groupe(s) fonctionnel(s) thiol sont des composés bien connus, qui ont des applications de plus en plus nombreuses. Une de ces applications est la déformation (frisage et défrisage) permanente des cheveux, qui consiste, dans un premier temps, à réaliser l'ouverture des liaisons disulfure (S-S) des unités cystine de la kératine à l'aide d'une composition contenant au moins un composé organique porteur de groupe fonctionnel thiol agissant comme réducteur (étape de réduction), ce qui permet de conférer aux cheveux la forme que l'on souhaite ; puis, après avoir rincé la chevelure, à reconstituer, dans un second temps, lesdites liaisons disulfure en appliquant, sur les cheveux, une composition oxydante (étape d'oxydation, dite aussi de fixation), de façon à fixer les cheveux dans la forme qui leur a été donnée ; dans ce but, on utilise particulièrement l'acide thioglycolique, l'acide thiolactique ou leurs mélanges, ou encore les esters de ces acides, par exemple les monothioglycolates de glycérol ou de glycol, ou aussi la cystéine ou la cystéamine. Les acides thioglycolique ou thiolactique et leurs sels sont également utilisés dans des laits et crèmes dépilatoires. Les acides thioglycolique et thiolactique ainsi que la cystéine sont aussi employés comme produits intermédiaires dans la fabrication de produits pharmaceutiques.

Malheureusement, si, en général, les composés porteurs d'un groupe fonctionnel thiol ont, à l'état pur, une odeur qui n'est pas désagréable, ils contiennent toujours, en pratique, des composés sulfurés tels que l'hydrogène sulfuré et des mercaptans de faible poids moléculaire, en particulier le méthanethiol ou l'éthanethiol, qui ont une odeur nauséabonde particulièrement désagréable. Il suffit de très faibles quantités de ces composés sulfurés pour que l'on perçoive leur présence à l'odorat, le nez étant, dans ce cas, le meilleur instrument de détection. Dans la suite de la description et dans les revendications, ces composés sulfurés seront désignés par le terme "composés malodorants".

La présence de ces composés malodorants est liée à divers processus mal connus de décomposition des composés porteurs d'un groupe thiol, en particulier par oxydation. La formation de ces composés malodorants peut être suivie au cours du temps par différentes techniques analytiques, en particulier par la méthode dite du "head space" en chromatographie gazeuse qui est, par exemple, décrite dans "Applied Headspace Gas Chromatography, B. KOLB, Editions HEYDEN, 1980".

Dans les différentes applications des composés porteurs d'un groupe fonctionnel thiol, et plus particulièrement dans leurs applications cosmétiques, l'odeur dégagée par les produits mis en oeuvre constitue une réelle gêne pour les utilisateurs. On a essayé de masquer l'odeur par des parfums, mais, en général, l'odeur est trop puissante pour pouvoir être masquée de façon significative.

On a donc cherché à éliminer les composés malodorants. Dans ce but, on a déjà proposé, dans la demande de brevet japonais publiée sous le n° 84-027 866, de désodoriser l'acide thioglycolique, pur ou en solution aqueuse, par extraction à l'aide d'un hydrocarbure non aromatique en C₄-C₈. Etant donné que, pour effectuer l'extraction, il est nécessaire d'utiliser une installation relativement volumineuse, il est pratiquement impossible de désodoriser les composés porteurs de groupe fonctionnel thiol juste avant leur utilisation de sorte que leur stockage est, en pratique, obligatoire. Or, on a constaté que, si ce procédé permet d'obtenir un acide désodorisé, l'effet de désodorisation obtenu n'est pas durable car les composés malodorants se reforment au cours du stockage, en particulier en présence d'oxygène, et même, dans certains cas, l'odeur revient à un niveau supérieur au niveau initial.

Il est donc nécessaire de trouver un procédé d'élimination des composés malodorants qui permette d'effectuer cette élimination au fur et à mesure de la formation desdits composés de façon à avoir, au moment de l'utilisation, un composé portant au moins un groupe fonctionnel thiol, qui soit désodorisé même après un stockage prolongé.

Il est connu d'éliminer, dans des gaz, les composés malodorants en cause à l'aide de substances, qui réagissent avec lesdits composés malodorants en donnant des composés qui ne sont pas gênants. Ces composés, comme décrit dans la demande de brevet japonais publiée sous le n° 88-264 139, peuvent être des oxydes d'argent, éventuellement mélangés à des oxydes de Co, Mn ou Cu. De façon connue, ces substances chimiques peuvent être contenues dans une membrane semi-perméable qui est traversée par le courant gazeux à purifier : par exemple, la demande de brevet japonais publiée sous le n° 88-023 708 décrit l'utilisation d'une membrane poreuse imprégnée d'un complexe métallique de phtalocyanine et traitée par le nitrate de cuivre.

Il est, par ailleurs, connu par FR-A 2 152 829 de dépolluer un courant d'air en faisant passer le gaz à travers une structure constituée par deux membranes semi-perméables, c'est-à-dire perméables aux gaz et aux vapeurs mais imperméables aux liquides, l'espace entre les deux membranes semi-perméables, contenant un agent capable de réagir avec le polluant. Selon ce document, la membrane semi-perméable sert donc essentiellement à maintenir en place l'agent capable de réagir avec le polluant et à empêcher qu'il ne soit entraîné par l'air. De plus, l'air ne contient pas de dérivés actifs porteurs de groupe(s) fonctionnel(s) thiol, que l'on souhaiterait ne pas faire réagir avec l'agent de rétention du polluant : il n'y a donc pas à séparer deux dérivés de thiol.

Il est également connu, par EP-A-0 488 665, un procédé dans lequel on met en contact une formulation liquide avec une membrane semi-perméable et ensuite les composés ayant traversé la membrane avec des substances chimiques réagissant avec ces composés.

Selon la présente invention, on a trouvé un procédé de désodorisation de composé(s) porteur(s) d'un groupe thiol, procédé qui permet d'éliminer de façon sélective les composés malodorants, sans que les réactifs utilisés pour éliminer lesdits composés malodorants n'aient une action sur le(s) composé(s) porteur(s) de groupe thiol traité(s) ; selon ce procédé, on met en oeuvre un système, qui est en contact avec ledit composé porteur d'un groupe thiol sous forme liquide, c'est-à-dire soit sous forme de liquide pur, soit sous forme de solution.

La présente invention a donc pour objet un procédé de désodorisation pour éliminer les composés malodorants présents dans ou formés à partir d'une formulation liquide contenant au moins un composé porteur d'un groupe fonctionnel thiol de formule :

HS―A―Y―B (I)

formule dans laquelle Y représente -COO- ou -NH- et
a) lorsque Y désigne -COO- :
   - A représente :
      - le radical divalent : -(CH₂)ₙ où n est 1 ou 2
      - le radical divalent : 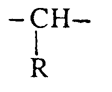 où R représente un radical alkyle, linéaire ou ramifié, en C₁-C₃ et
   - B représente les radicaux -H ; -CH₂-CH₂OH ; -CH₂-CHOH - CH₂OH ; 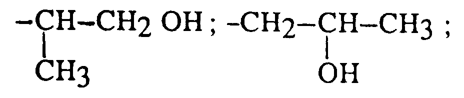
b) lorsque Y représente -NH-
   - A représente
      -CH₂-CH₂- ou 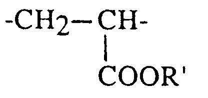 R' représentant -H, un radical méthyle ou un radical éthyle
   - B représente -H ou, lorsque A représente -CH₂-CH₂-,
      B représente également un radical -CO-R'',
      R'' représentant un radical alkyle, linéaire ou ramifié, en C₁-C₄ ;
caractérisé par le fait que :
1) on met en contact la formulation liquide avec une membrane inerte, perméable aux composés malodorants et imperméable au(x) composé(s) de formule (I); et
2) on met les composés malodorants ayant traversé la membrane en contact avec au moins une substance d' adsorption physique à grande surface spécifique fixant lesdits composés malodorants.

Selon la présente invention, on peut mélanger la substance d'adsorption physique à au moins une substance chimique réagissant avec lesdits composés malodorants.

Dans ce procédé, la membrane sert donc, à la fois, à séparer de façon sélective les composés malodorants par rapport au(x) composé(s) de formule (I) et à maintenir les substances d' adsorption physique éventuellement mélangées à une substance chimique réagissant avec les composés malodorants.

La formulation liquide contenant au moins un composé de formule (I) peut contenir le composé de formule (I) en tant que tel, s'il est à l'état liquide dans les conditions de température et de pression appliquées à la formulation, ou le composé de formule (I) sous forme de solution, notamment de solution aqueuse.

Le composé de formule (I) est avantageusement choisi dans le groupe formé par l'acide thioglycolique (ou acide mercaptoacétique), l'acide thiolactique (ou acide mercapto-2 propionique), l'acide mercapto-3 propionique, le monothioglycolate de glycérol, le mercapto-2 propionate de glycérol, un mélange azéotrope de thioglycolate d'hydroxy-2 propyle et de thioglycolate d'hydroxy-2 méthyl-1 éthyle (tel que décrit dans FR-A 2 679 448), la cystéamine (ou amino-2 éthanethiol) et ses dérivés N-acylés, le radical acyle comportant 2 à 5 atomes de carbone, la cystéine et les cystéinates de méthyle et d'éthyle. On préfère les acides thioglycolique et thiolactique.

La membrane utilisée selon l'invention est avantageusement en un matériau à caractère hydrophobe choisi dans le groupe formé par le polytétrafluoroéthylène, par exemple celui commercialisé sous la dénomination "TEFLON" par la société "DUPONT DE NEMOURS", le polyéthylène, en particulier le polyéthylène basse densité, le polypropylène, le polystyrène et les copolymères de butadiène et de styrène, en particulier celui commercialisé sous la dénomination "STYROLUX" par la société "BASF".

La substance d'adsorption physique des composés malodorants est, avantageusement, choisie dans le groupe formé par les charbons actifs, les gels de silice, l'alumine, les tamis moléculaires, les copolymères styrène-divinylbenzène ou styrène-éthénylbenzène, en particulier celui commercialisé sous la dénomination "PORAPAK" par la société "WATERS", et les polyphénylèneoxydes, en particulier celui commercialisé sous la dénomination "TENAX" par la société "AKZO".

La (les) substance(s) chimique(s) réagissant éventuellement avec les composés malodorants est (sont) choisie(s) de façon à donner par réaction des composés qui n'ont plus d'odeur. Elle(s) peu(ven)t être avantageusement choisie(s) dans le groupe formé par un métal finement divisé, tel que le cuivre, le zinc ou l'argent, un oxyde métallique finiment divisé, tel que Cu₂O, MnO₂, ZnO ou les oxydes d'argent et une solution, notamment aqueuse, de sel(s) métallique(s), tel que l'acétate de cadmium, l'acétate de plomb, le sulfate de cuivre ou le sulfate de fer.

L'ensemble membrane-substance(s) d'adsorption physique et, éventuellement, substance(s) chimique(s) utilisé dans le procédé de la présente invention peut se présenter sous différentes formes.

Selon un premier mode de réalisation avantageux de cet ensemble, la membrane double la face interne de la paroi du récipient contenant la formulation à désodoriser ; la (les) substance(s) d'adsorption physique et, éventuellement, la (les) substance(s) chimique(s) réagissante(s) destinée(s) à agir sur les composés malodorants sont alors disposées entre la membrane et la paroi dudit récipient.

Selon une variante de ce mode de réalisation, la membrane constitue la face interne de la paroi d'un bonnet destiné à recouvrir une chevelure traitée par une formulation générant des composés malodorants ; la (les) substance(s) d'adsorption physique et, éventuellement, la (les) substance(s) chimique(s) réagissante(s) est (sont) disposée(s) entre ladite membrane et la face externe du bonnet. Un tel bonnet évite les mauvaises odeurs au cours d'une permanente de cheveux car les composants malodorants sont piégés a l'intérieur du bonnet.

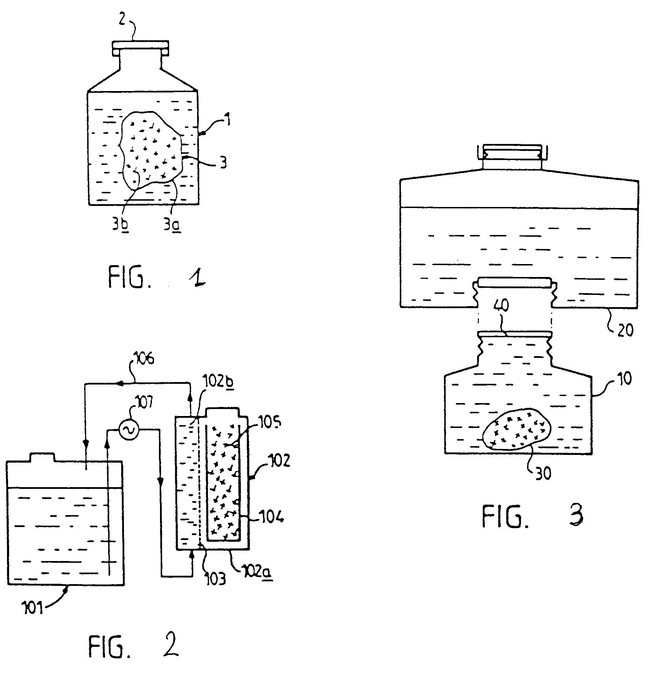

Selon un deuxième mode de réalisation, la membrane forme un sac, qui est rempli de substance(s) d'adsorption physique et, éventuellement, de substance(s) chimique(s) destinée(s) à agir sur les composés malodorants ; un ou plusieurs de ces sacs est (sont) alors plongé(s) dans le récipient où est conditionnée la formulation liquide à désodoriser. Ces sacs peuvent avoir des formes variables, de section circulaire, carrée, rectangulaire ; ils peuvent constituer des tubes permettant le passage de la formulation liquide. Un ensemble de ce type est illustré schématiquement sur la figure 1. Sur cette figure 1, le récipient est un flacon désigné par la référence 1 ; il est scellé de façon étanche par un opercule 2 et il contient un sachet 3 constitué par une membrane 3a hermétiquement soudée, qui contient au moins une substance d'adsorption physique 3b et, éventuellement, une substance chimique réagissante.

Selon un troisième mode de réalisation, la (les) substance(s) d'adsorption physique et, éventuellement, la (les) substance(s) chimique(s) réagissante(s) est (sont) sous forme particulaire et la membrane constitue un revêtement autour de chacune desdites particules. Les particules de substance(s) enrobées sont introduites dans le récipient contenant la formulation liquide à désodoriser.

Selon un quatrième mode de réalisation, la membrane ainsi que la (les) substance(s) d'adsorption physique et, éventuellement, la (les) substance(s) chimique(s) réagissante(s) sont situées à l'extérieur du récipient contenant la formulation à désodoriser et un dispositif de circulation permet de mettre en contact ladite formulation liquide avec la membrane. Un ensemble de ce type est illustré schématiquement sur la figure 2. L'installation illustrée sur la figure 2 comporte un réservoir 101 contenant la formulation liquide à désodoriser et une unité de désodorisation 102 séparée en deux compartiments 102a, 102b par une membrane 103. Le compartiment 102a renferme un panier amovible 104 contenant la (les) substance(s) d'adsorption physique 105 et, éventuellement, la (les) substance(s) chimique(s) réagissante(s). L'autre compartiment 102b est relié par l'intermédiaire de conduites 106 au réservoir 101, de façon que le liquide contenu dans le réservoir 101 puisse circuler en circuit fermé du réservoir 101 au compartiment 102b et vice-versa ; la circulation est assurée par une pompe 107 qui génère une légère surpression du côté Je la membrane 103 où se trouve le compartiment 102b, grâce à laquelle les composés malodorants passent du compartiment 102b vers le compartiment 102a. De préférence, le réservoir 101 est maintenu sous atmosphère inerte.

Selon l'invention, l'ensemble (membrane/substance(s) d'adsorption physique et, éventuellement, substance(s) chimique(s) réagissante(s)) peut être en contact avec le(s) composé(s) de formule (I) pendant tout le temps de stockage. Cet ensemble élimine, par conséquent, les composés malodorants au fur et à mesure de leur formation et, au moment de l'utilisation, la formulation contenant le(s) composé(s) de formule (I) se trouve toujours sous forme désodorisée.

La présente invention a également pour objet la formulation liquide de composé(s) de formule (I) désodorisée obtenue selon le procédé décrit ci-dessus.

La formulation de composé(s) de formule (I) désodorisée peut être utilisée dans toutes les applications industrielles connues pour ces composés. Les composés de formule (I) sont généralement associés à d'autres ingrédients dans la formulation, en fonction de l'application. L'efficacité de la désodorisation permet de les utiliser, sans donner d'odeur désagréable, dans toute composition qui ne doit pas avoir d'odeur désagréable et dont les autres ingrédients n'ont pas d'odeur désagréable.

Cependant, en particulier lorsque la composition utilisée contient des ingrédients susceptibles de faire perdre le bénéfice de la désodorisation en provoquant la formation de composés malodorants, il peut être avantageux de conditionner séparément le(s) composé(s) de formule (I) et certains des ingrédients de la composition finale désirée ; on prépare alors au dernier moment la quantité de composition nécessaire à l'application. Un conditionnement de ce type est illustré, de façon schématique, sur la figure 3. Le conditionnement illustré comporte deux flacons 10, 20 : le premier flacon 10 contient un composé de formule (I) et un sachet 30 formé par une membrane hermétiquement fermée contenant au moins une substance d'adsorption physique et, éventuellement, au moins une substance chimique réagissante. Le flacon 20 contient les autres ingrédients de la composition à préparer. Le flacon 10 est fermé par un opercule 40 et un dispositif porté par le flacon 20 permet de déchirer cet opercule 40 et d'introduire le contenu du flacon 10 dans le flacon 20 pour préparer au moment de l'emploi la composition désirée. Un système à deux flacons utilisable selon la présente invention a, par exemple, été décrit dans EP-A 0 528 707.

Les exemples donnés ci-après, à titre illustratif et non limitatif, permettront de mieux comprendre l'invention.

### EXEMPLE 1

Dans un flacon de 60 ml de capacité, on a introduit un ensemble de désodorisation constitué par un sachet en un copolymère butadiène-styrène, commercialisé sous la dénomination "STYROLUX KR 2688" par la société "BASF", de 40 µm d'épaisseur et de 50 cm² de surface. Le sachet contient 2,0 g de charbon actif commercialisé sous la dénomination "ACTICARBONE ACF3" par la société "CECA". On introduit ensuite dans le flacon 30 ml d'une solution aqueuse d'acide thioglycolique à 184 g/l (2 M). Le flacon est scellé. Il contient alors 30 ml d'air.

On a préparé simultanément un flacon identique mais ne contenant pas de sachet de désodorisation. On a stocké les deux flacons à température ambiante.

On a mesuré par chromatographie en phase gazeuse du "head space" la teneur en hydrogène sulfuré. On a constaté qu'au bout de 3 jours, la teneur en H₂S dans le flacon contenant le sachet de désodorisation est 4,5 % de celle dans le flacon ne contenant pas de sachet de désodorisation ; après 10 jours de stockage, cette teneur n'est plus que de 2 % et après 20 jours, elles n'est plus que de 0,1 %.

### EXEMPLE 2

Dans un flacon de 60 ml, on a introduit un sachet hermétiquement scellé en un copolymère butadiène-styrène commercialisé par la société "BASF" sous la dénomination "STYROLUX KR 2688", de 40 µm d'épaisseur et de 50 cm² de surface, contenant 1,5 g d'un mélange de charbon actif, de noir animal et d'oxyde cuivreux à 97 % en poids, le rapport en poids (charbon actif-noir animal/oxyde cuivreux) étant de 2/1. On ajoute ensuite 30 ml d'une solution d'acide thioglycolique à 184 g/l (2 M). Le flacon est scellé ; il contient 30 ml d'air.

On prépare simultanément un flacon identique mais ne contenant pas de sachet de désodorisation. Les deux flacons sont conservés à température ambiante.

On a mesuré par chromatographie en phase gazeuse du "head space" la teneur en H₂S dans chacun des deux flacons. On a constaté, qu'au bout de 3 jours, la teneur en H₂S dans le flacon contenant le sachet de désodorisation est de 7 % de celle dans l'unité ne contenant pas de sachet de désodorisation, qu'elle n'est plus que de 2 % au bout de 10 jours et de 0,15 % au bout de 20 jours.

### EXEMPLE 3

Dans un flacon de 60 ml de capacité, on introduit un sachet en polyéthylène basse densité de 20 µm d'épaisseur et de 50 cm² de surface contenant 1,8 g d'alumine activée commercialisée par la société "PROCATALYSE". On introduit ensuite 30 ml d'une solution d'acide thioglycolique à 184 g/l (2 M). Le flacon est scellé ; il contient environ 30 ml d'air.

On prépare, comme dans les exemples 1 et 2, un flacon témoin et on mesure dans les deux flacons la teneur en H₂S après stockage à température ambiante. On constate qu'au bout de 3 jours, la teneur en H₂S dans le flacon contenant le sachet de désodorisation n'est que 22 % de celle dans le flacon témoin, qu'après 10 jours, elle n'est plus que de 2 % et après 20 jours elle est de, 2 % également.

### EXEMPLE 4

On prépare une composition réductrice de déformation permanente des cheveux en procédant au mélange des ingrédients suivants :

| | |
|---|---|
| - Acide thioglycolique | 9,1 g |
| - Mélange de cocamidopropylbétaïne et de laurate de glycéryle vendu sous la dénomination "TEGOBETAINE HS" par la société "GOLDSCHMIDT" en solution à 30 % de matières actives (MA) | 0,4 g (MA) |
| - Alcool oléique oxyéthyléné à 20 moles d'oxyde d'éthylène | 0,8 g |
| - Eau qsp | 100 g |

On applique environ 75 ml de cette solution sur des cheveux mouillés préalablement enroulés sur des rouleaux.

On place sur la chevelure un bonnet constitué d'une double paroi mince en polyéthylène basse densité, chaque paroi ayant une épaisseur de 20 µm et une surface de 1 300 cm². Entre les deux parois sont uniformément répartis 2 g d'un mélange constitué de 1,4 g de charbon actif vendu sous la dénomination "AS 1544" par la société "CECA" et de 0,6 g d'oxyde cuivreux. Le bonnet est muni d'une fixation.

On laisse agir la composition pendant 15 minutes, après avoir recouvert la chevelure avec le bonnet. On constate que le bonnet évite les mauvaises odeurs au cours de la phase de réduction de la permanente.

On rince ensuite abondamment à l'eau puis on applique la composition oxydante suivante :

| | |
|---|---|
| - Eau oxygénée | 1,5 g |
| - Lauryléther sulfate de sodium oxyéthyléné à 2 moles d'oxyde d'éthylène | 3,75 g |
| - Acide citrique | 0,5 g |
| - Hydrogénophosphate de sodium | 0,5 g |
| - Parfum | 0,3 g |
| - Eau déminéralisée qsp | 100 g |

On laisse agir la composition oxydante pendant environ 5 minutes, puis on enlève les rouleaux et on rince abondamment la chevelure à l'eau. Après séchage sous casque, les cheveux présentent de belles boucles.

## Revendications

1. Procédé de désodorisation pour éliminer les composés malodorants présents dans ou formés à partir d'une formulation liquide contenant au moins un composé porteur d'un groupe fonctionnel thiol de formule :
HS―A―Y―B (I)
formule dans laquelle Y représente -COO- ou -NH- et
a) lorsque Y désigne -COO- :
• A représente :
- le radical divalent : -(CH₂)ₙ où n est 1 ou 2
- le radical divalent : 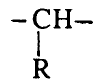 où R représente un radical alkyle, linéaire ou ramifié, en C₁-C₃ et
• B représente les radicaux -H ; -CH₂-CH₂OH ; -CH₂-CHOH - CH₂OH ; 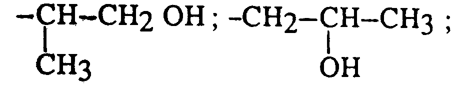
b) lorsque Y représente -NH-
• A représente
-CH₂-CH₂- ou 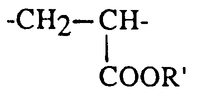 R' représentant -H, un radical méthyle ou un radical éthyle
• B représente -H ou, lorsque A représente -CH₂-CH₂-,
B représente également un radical -CO-R'',
R'' représentant un radical alkyle, linéaire ou ramifié, en C₁-C₄,
caractérisé par le fait que :
1) on met en contact la formulation liquide avec une membrane inerte, perméable aux composés malodorants et imperméable au(x) composé(s) de formule (I) ; et
2) on met les composés malodorants ayant traversé la membrane en contact avec au moins une substance d'adsorption physique à une grande surface spécifique fixant lesdits composés malodorants.

2. Procédé selon la revendication 1, caractérisé par le fait que l'on met en contact les composés malodorants ayant traversé la membrane avec au moins une substance d'adsorption physique et avec au moins une substance chimique réagissant avec les composés malodorants.

3. Procédé selon l'une des revendications 1 ou 2, caractérisé par le fait que l'on choisit le composé de formule (I) dans le groupe formé par l'acide thioglycolique, l'acide thiolactique, l'acide mercapto-3 propionique, le monothioglycolate de glycérol, le mercapto-2 propionate de glycérol, un mélange azéotrope de thioglycolate d'hydroxy-2 propyle et de thioglycolate d'hydroxy-2 méthyl-1 éthyle, la cystéamine et ses dérivés N-acylés, le radical acyle comportant 2 à 5 atomes de carbone, la cystéine et les cystéinates de méthyle et d'éthyle.

4. Procédé selon l'une des revendications 1 à 3, caractérisé par le fait que l'on choisit le matériau de la membrane dans le groupe formé par le polytétrafluoroéthylène, le polyéthylène, le polypropylène, le polystyrène et les copolymères de butadiène et de styrène.

5. Procédé selon l'une des revendications 1 à 4, caractérisé par le fait que l'on choisit la substance d'adsorption physique dans le groupe formé par les charbons actifs, les gels de silice, l'alumine, les tamis moléculaires, les copolymères styrène-divinylbenzène ou styrène-éthénylbenzène et les polyphénylène oxydes.

6. Procédé selon l'une des revendications 2 à 5, caractérisé par le fait que l'on choisit la substance chimique réagissant avec les composés malodorants dans le groupe formé par le cuivre, le zinc, l'argent, Cu₂O, MnO₂, ZnO, les oxydes d'argent, tous sous forme finement divisée, et les solutions aqueuses d'acétate de cadmium, d'acétate de plomb, de sulfate de cuivre ou de sulfate de fer.

7. Procédé selon l'une des revendications 1 à 6, caractérisé par le fait que la membrane double la face interne de la paroi du récipient contenant la formulation à désodoriser, la (les) substance(s) d'adsorption physique destinée(s) à agir sur les composés malodorants et, éventuellement, la (les) substance(s) chimique(s) réagissante(s) est (sont) disposée(s) entre la membrane et la paroi du récipient.

8. Procédé selon l'une des revendications 1 à 6, caractérisé par le fait que la membrane forme au moins un sac, qui contient la (les) substance(s) d'adsorption physique et, éventuellement, la (les) substance(s) chimique(s) réagissante(s) destinée(s) à agir sur les composés malodorants, et que l'on plonge au moins un tel sac dans la formulation liquide à désodoriser.

9. Procédé selon l'une des revendications 1 à 6, caractérisé par le fait que les substance(s) d'adsorption physique et, éventuellement, la (les) substance(s) chimique(s) réagissante(s) destinée(s) à agir sur les composés malodorants est (sont) sous forme particulaire et que l'on enrobe chacune desdites particules d'un revêtement constituant la membrane, les particules enrobées étant introduites dans le récipient contenant la formulation à désodoriser.

10. Procédé selon l'une des revendications 1 à 6, caractérisé par le fait que l'on dispose la membrane ainsi que la (les) substance(s) d'adsorption physique et, éventuellement, la (les) substance(s) chimique(s) réagissante(s) destinée(s) à agir sur les composés malodorants à l'extérieur du récipient contenant la formulation liquide à désodoriser et l'on met en contact ladite formulation et la membrane au moyen d'un dispositif de circulation.

11. Procédé selon la revendication 1, caractérisé par le fait que la membrane constitue la face interne de la paroi d'un bonnet destiné à recouvrir une chevelure traitée par la formulation qui contient au moins un composé de formule (I), la (les) substance(s) d'adsorption physique et, éventuellement, la (les) substance(s) chimique(s) réagissante(s) étant disposée(s) entre ladite membrane et la face externe du bonnet.

## Claims

1. Method of deodorizing in order to remove the malodorous compounds which are present in or formed from a liquid formulation containing at least one compound bearing a thiol functional group, of formula:
HS―A―Y―B (1)
in which formula Y represents -COO- or -NH- and
a) when Y denotes -COO-:
• A represents:
- the divalent radical: -(CH₂)ₙ where n is 1 or 2
- the divalent radical: 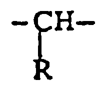 where R represents a linear or branched C₁-C₃ alkyl radical, and
• B represents the radicals -H; -CH₂-CH₂OH; -CH₂-CHOH-CH₂OH; 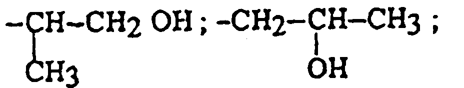
b) when Y represents -NH-
• A represents:
-CH₂-CH₂- or 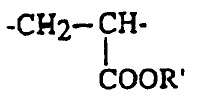 R' representing -H, a methyl radical or an ethyl radical
• B represents -H or, when A represents -CH₂-CH₂-,
B also represents a radical -CO-R'',
R'' representing a linear or branched C₁-C₄ alkyl radical,
characterized in that:
1) the liquid formulation is placed in contact with an inert membrane which is permeable to the malodorous compounds and impermeable to the compound(s) of formula (I); and
2) the malodorous compounds which have passed through the membrane are placed in contact with at least one physical adsorption substance having a large specific surface area which binds the said malodorous compounds.

2. Method according to Claim 1, characterized in that the malodorous compounds which have passed through the membrane are placed in contact with at least one physical adsorption substance and with at least one chemical substance which reacts with the malodorous compounds.

3. Method according to either of Claims 1 and 2, characterized in that the compound of formula (I) is chosen from the group formed by thioglycolic acid, thiolactic acid, 3-mercaptopropionic acid, glycerol monothioglycolate, glycerol 2-mercaptopropionate, an azeotropic mixture of 2-hydroxypropyl thioglycolate and 2-hydroxy-1-methylethyl thioglycolate, cysteamine and its N-acylated derivatives, the acyl radical containing 2 to 5 carbon atoms, cysteine and the methyl and ethyl cysteinates.

4. Method according to one of Claims 1 to 3, characterized in that the membrane material is chosen from the group formed by polytetrafluoroethylene, polyethylene, polypropylene, polystyrene and copolymers of butadiene and styrene.

5. Method according to one of Claims 1 to 4, characterized in that the physical adsorption substance is chosen from the group formed by active charcoals, silica gels, alumina, molecular sieves, styrene-divinylbenzene or styrene-ethenylbenzene copolymers and polyphenylene oxides.

6. Method according to one of Claims 2 to 5, characterized in that the chemical substance reacting with the malodorous compounds is chosen from the group formed by copper, zinc, silver, Cu₂O, MnO₂, ZnO, silver oxides, all in finely divided form, and the aqueous solutions of cadmium acetate, lead acetate, copper sulphate or iron sulphate.

7. Method according to one of Claims 1 to 6, characterized in that the membrane lines the inner face of the wall of the receptacle containing the formulation to be deodorized, the physical adsorption substance(s) intended to act on the malodorous compounds and the optionally, reacting chemical substance(s) is (are) arranged between the membrane and the wall of the receptacle.

8. Method according to one of Claims 1 to 6, characterized in that the membrane forms at least one bag, which contains the physical adsorption substance(s) and, optionally, the reacting chemical substance(s) intended to act on the malodorous compounds, and in that at least one such bag is immersed in the liquid formulation to be deodorized.

9. Method according to one of Claims 1 to 6, characterized in that the physical adsorption substance(s) and, optionally, the reacting chemical substance(s) intended to act on the malodorous compounds is (are) in particulate form and in that each of the said particles is coated with covering constituting the membrane, the coated particles being introduced into the receptacle containing the formulation to be deodorized.

10. Method according to one of Claims 1 to 6, characterized in that the membrane, as well as the physical adsorption substance(s) and, optionally, the reacting chemical substance(s) intended to act on the malodorous compounds are arranged outside the receptacle containing the liquid formulation to be deodorized, and the said formulation and the membrane are placed in contact by means of a circulation device.

11. Method according to Claim 1, characterized in that the membrane constitutes the inner face of the wall of a cap intended to cover a head of hair treated with the formulation which contains at least one compound of formula (I), the physical adsorption substance(s) and, optionally, the reacting chemical substance(s) being arranged between the said membrane and the outer face or the cap.

## Patentansprüche

1. Desodorierungsverfahren zum Entfernen von unangenehm riechenden Verbindungen, die in einer flüssigen Formulierung vorhanden sind oder von dieser gebildet werden, welche wenigstens eine Verbindung enthält, die eine funktionelle Thiolgruppe der Formel
HS - A - Y - B (1)
trägt, worin Y für -COO- oder -NH- steht und,
a) falls Y für -COO- steht,
• A für
- den divalenten Rest -(CH₂)ₙ, worin n für 1 oder 2 steht,
- den divalenten Rest 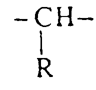
- steht, worin R für einen linearen oder verzweigten C₁-C₃-Alkylrest steht, und
• B für die Reste -H; -CH₂-CH₂OH; -CH₂-CHOH-CH₂OH; 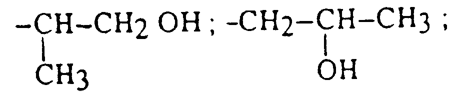 steht,
b) falls Y für -NH- steht,
• A für
-CH₂-CH₂- oder 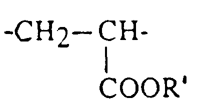 steht, wobei R' für -H, einen Methylrest oder einen Ethylrest steht,
• B für -H oder, falls A für -CH₂-CH₂- steht,
B auch für einen Rest -CO-R'' steht, wobei
R'' für einen linearen oder verzweigten C₁-C₄-Alkylrest steht,
dadurch gekennzeichnet, daß
1) man die flüssige Formulierung mit einer inerten, für die unangenehm riechenden Verbindungen permeablen und für die Verbindung(en) der Formel (I) impermeablen Membran in Kontakt bringt; und
2) man die unangenehm riechenden Verbindungen, welche die Membran durchquert haben, mit wenigstens einer Substanz zur physikalischen Adsorption an einer großen spezifischen Oberfläche in Kontakt bringt, wobei die unangenehm riechenden Verbindungen fixiert werden.

2. Verfahren nach Anspruch 1, dadurch gekennzeichnet, daß die unangenehm riechenden Verbindungen, welche die Membran durchquert haben, mit wenigstens einer Substanz zur physikalischen Adsorption und mit wenigstens einer chemischen Substanz, die mit den unangenehm riechenden Verbindungen reagiert, in Kontakt bringt.

3. Verfahren nach einem der Ansprüche 1 oder 2, dadurch gekennzeichnet, daß die Verbindung der Formel (I) ausgewählt ist unter Thioglycolsäure, Thiomilchsäure, 3-Mercaptopropionsäure, Glycerinmonothioglycolat, Glycerin-2-mercaptopropionat, ein azeotropes Gemisch aus (2-Hydroxypropyl)thioglycolat und (2-Hydroxy-1-methylethyl)thioglycolat, Cysteamin und dessen N-acylierte Derivate, wobei der Acylrest 2 bis 5 Kohlenstoffatome umfaßt, Cystein und Methyl- und Ethylcysteinat.

4. Verfahren nach einem der Ansprüche 1 bis 3, dadurch gekennzeichnet, daß das Membranmaterial ausgewählt ist unter Polytetrafluorethylen, Polyethylen, Polypropylen, Polystyrol und Copolymeren aus Butadien und Styrol.

5. Verfahren nach einem der Ansprüche 1 bis 4, dadurch gekennzeichnet, daß die Substanz zur physikalischen Adsorption ausgewählt ist unter Aktivkohlen, Kieselgelen, Tonerde, Molekularsieben, Styrol-Divinylbenzol- oder Styrol-Ethenylbenzol-Copolymeren und Polyphenylenoxiden.

6. Verfahren nach einem der Ansprüche 2 bis 5, dadurch gekennzeichnet, daß die mit den unangenehm riechenden Verbindungen reagierende chemische Substanz ausgewählt ist unter Kupfer, Zink, Silber, Cu₂O, MnO₂, ZnO, Silberoxiden, alle in feinverteilter Form, und wäßrigen Lösungen von Cadmiumacetat, Bleiacetat, Kupfersulfat oder Eisensulfat.

7. Verfahren nach einem der Ansprüche 1 bis 6, dadurch gekennzeichnet, daß die Membran die Wandinnenfläche des die zu desodorierende Formulierung enthaltenden Behälters auskleidet, und die zur Wirkung auf die unangenehm riechenden Verbindungen bestimmte(n) Substanz(en) zur physikalischen Adsorption und gegebenenfalls die reagierende(n) chemische(n) Substanz(en) zwischen der Membran und der Behälterwand angeordnet sind.

8. Verfahren nach einem der Ansprüche 1 bis 6, dadurch gekennzeichnet, daß die Membran wenigstens einen Sack bildet, der die Substanz(en) zur physikalischen Adsorption und gegebenenfalls die reagierende(n) chemische(n) Substanz(en), welche auf die unangenehm riechenden Verbindungen wirken soll(en), enthält, und man wenigstens einen solchen Sack in die zu desodorierende flüssige Formulierung taucht.

9. Verfahren nach einem der Ansprüche 1 bis 6, dadurch gekennzeichnet, daß die Substanz(en) zur physikalischen Adsorption und gegebenenfalls die reagierende(n) chemische(n) Substanz(en), welche auf die unangenehm riechenden Verbindungen wirken soll(en), in Form von Teilchen vorliegt(en) und daß man jedes Teilchen mit einem die Membran bildenden Überzug umhüllt, wobei die umhüllten Teilchen in den die zu desodorierende Formulierung enthaltenen Behälter eingebracht wird.

10. Verfahren nach einem der Ansprüche 1 bis 6, dadurch gekennzeichnet, daß man die Membran sowie die Substanz(en) zur physikalischen Adsorption und gegebenenfalls die reagierende(n) chemische(n) Substanz(en), welche auf die unangenehm riechenden Verbindungen wirken soll(en), außerhalb des Behälters, der die zu desodorierende flüssige Formulierung enthält, anordnet und man die Formulierung und die Membran mit Hilfe einer Umlaufvorrichtung in Kontakt bringt.

11. Verfahren nach Anspruch 1, dadurch gekennzeichnet, daß die Membran die Wandinnenfläche einer Kappe bildet, welche das Haar bedecken soll, das mit der Formulierung, die wenigstens eine Verbindung der Formel (I) enthält, behandelt worden ist, wobei die Substanz(en) zur physikalischen Adsorption und gegebenenfalls die reagierende(n) chemische(n) Substanz(en) zwischen der Membran und der Kappenaußenfläche angeordnet sind.
